# EUROPEAN PATENT APPLICATION

(11) **EP 3 033 988 A1**
(43) Date of publication of application: **22.06.2016**
(21) Application number: 15160579.7
(22) Date of filing: 24.03.2015
(51) Int. Cl.: A61B 5/00

(54) **WEARABLE MEDICAL SENSOR SYSTEM**

(30) Priority: 16.12.2014 TW 103143966
(71) Applicant: Kinpo Electronics, Inc., New Taipei City 22201 (TW); Cal-Comp Electronics & Communications Company Limited, New Taipei City 22201 (TW)
(72) Inventor: Haraikawa, Koichi, Shenkeng Dist., New Taipei City, 22201 (JP); Chien, Jen-Chien, 22201 Shenkeng Dist., New Taipei City (TW); Hsu, Tsai-Chieh, 22201 Shenkeng Dist., New Taipei City (TW); Liu, Chih-Wei, 22201 Shenkeng Dist., New Taipei City (TW)
(74) Representative: 2K Patentanwälte Blasberg Kewitz & Reichel

(57) **Abstract**

A wearable medical sensor system includes a piece of clothes (100) and an elastic bounding layer (200) attached in the clothes (100). The piece of clothes (100) includes a main body (110) with a shape symmetric on both sides with respect to an axis of symmetry (101) and a couple of sleeves (120) symmetrically connected to both sides of the main body (110). At least a part of the main body (110) is surrounded by the elastic bounding layer (200). First to Fifth electrodes (310/320/330/340/350) are attached on the elastic bounding layer (200) corresponding to the same surface of the clothes (100). The elastic bounding layer (200) is more stretchable and more contractible than the clothes (100).

## Description

### TECHNICAL FIELD

The technical field of the present disclosure relates to apparatuses used for measuring electrocardiograms, and more particularly to a wearable medical sensor system used for measuring the electrocardiograms.

### BACKGROUND

In conventional electrocardiography (ECG), there are a total of twelve leads including six leads on the frontal plane and six leads on the horizontal plane. After a doctor performs an electrocardiographic test to a patient, the doctor should immediately examine the electrocardiograms of these twelve leads and check whether there is any abnormal waveform or rhythm.

The conventional 12-lead ECG has the drawbacks of being too cumbersome and too time-consuming to review the leads one by one. Therefore, a 5-electrode electrocardiographic measurement system (or Holter system) was developed, and such system uses 5 electrode pads attached to specific positions of a patient's body to calculate the standard 12-lead electrocardiogram (ECG) signals by vectors in order to simplify the measurements. The advantage resides on that this system is able to measure the 12-lead ECG signals without using complicated electrode pads, so as to save the trouble and lower the level of difficulty of attaching the electrode pads. Compared with the standard 12-lead electrocardiographic measurement system, the 5-electrode electrocardiographic measurement system reduces discomfort and inconvenience during the wearing and measuring processes.

The conventional 5-electrode electrocardiographic measurement system (Holter system) still adopts conductive wires and electrode pads, and definitely affects the examinee to a certain extent, so that such system is only suitable for the use in occasional diagnoses, and the conductive wire may be shaken or moved to cause a signal shift. Obviously, the conventional Holter system is unsuitable for long-term monitoring or warning heart attack or disease.

In view of the aforementioned drawbacks of the prior art, the inventor of the present disclosure based on years of experience in the related industry to conduct extensive researches and experiments, and finally designed a feasible solution to overcome the drawbacks of the prior art.

### SUMMARY

Therefore, it is a primary objective of the present disclosure to provide a wearable medical sensor system used for measuring electrocardiograms.

To achieve the aforementioned and other objectives, the present disclosure provides a wearable medical sensor system, comprising a piece of clothes and an elastic bounding layer attached on an inner side of the piece of clothes, and the clothes includes a main body with a shape symmetric on both sides with respect to an axis of symmetry and a pair of sleeves symmetrically connected to both sides of the main body, and at least a part of the main body is surrounded by the elastic bounding layer, and a first electrode, a second electrode, a third electrode, a fourth electrode and a fifth electrode are attached on the elastic bounding layer and disposed on the same surface corresponsive to the clothes, and the elastic bounding layer 200 has a contractility greater than the contractility of the clothes, and the elastic bounding layer 200 has a stretchability greater than the stretchability of the clothes.

Preferably, the first electrode and the second electrode are disposed symmetrically on both sides of the axis of symmetry and arranged under a lower-edge connecting line of the junction of the main body and each sleeve junction. The third electrode and fourth electrode are arranged between an upper-edge connecting line and a lower-edge connecting line of the junction of the main body and each sleeve and disposed on both sides of the axis of symmetry respectively. The fifth electrode is disposed on a lower-edge connecting line of the junction of the main body and each sleeve junction and configured to be biased towards a side of the axis of symmetry. The third electrode and the fourth electrode are configured to be corresponsive to the sleeves respectively.

The first electrode, the second electrode, the third electrode, the fourth electrode and the fifth electrode preferably are conductive fabrics. The elastic bounding layer may further include a belt attached on the clothes. The elastic bounding layer may further include a vest attached on an inner side of the main body, and only a part of the elastic bounding layer is coupled to a part of the clothes. The elastic bounding layer is attached on an inner side of the main body and extended to an inner side of at least one sleeve.

The piece of clothes preferably includes an ECG signal processing device attached thereon and electrically coupled to the first electrode, the second electrode, the third electrode, the fourth electrode and the fifth electrode.

The present disclosure also provides a wearable medical sensor system comprising a piece of clothes and an elastic bounding layer spliced with the clothes, and the piece of clothes includes a main body with a shape symmetric on both sides with respect to an axis of symmetry and a pair of sleeves symmetrically connected to both sides of the main body respectively and surrounded by at least a part of the elastic bounding layer, and a first electrode, a second electrode, a third electrode, a fourth electrode and a fifth electrode are attached on the elastic bounding layer and configured on the same surface of the clothes, and the elastic bounding layer has a contractility greater than the contractility of the clothes, and the elastic bounding layer has a stretchability greater than the stretchability of the clothes.

The first electrode and the second electrode are disposed symmetrically on both sides of the axis of symmetry and arranged under a lower-edge connecting line of the junction of the main body and each sleeve junction, and the third electrode and the fourth electrode are arranged between an upper-edge connecting line and the lower-edge connecting line of the junction of the main body and each sleeve and disposed on both sides of the axis of symmetry respectively, and the fifth electrode is disposed on the lower-edge connecting line of the junction of the main body and each sleeve junction and configured to be biased towards a side of the axis of symmetry. The third electrode and the fourth electrode are configured to be corresponsive to the sleeves respectively.

Preferably, the first electrode, the second electrode, the third electrode, the fourth electrode and the fifth electrode are conductive fabrics. The elastic bounding layer may further include a belt spliced with the clothes, or an ECG signal processing device attached on the clothes and electrically coupled to the first electrode, the second electrode, the third electrode, the fourth electrode and the fifth electrode.

In the wearable medical sensor system of the present disclosure, the elastic bounding layer is connected to the electrodes and provided for fixing the electrodes at their relative positions, so that when a user wears the clothes, the elastic bounding layer fixes each electrode onto a predetermined measuring position of the user's body, so that the user can install the electrodes alone.

### BRIEF DESCRIPTION OF THE DRAWINGS

FIG. 1 is a schematic view of a wearable medical sensor system in accordance with a first preferred embodiment of the present disclosure;
FIG. 2 is a schematic view of a using status of a wearable medical sensor system in accordance with the first preferred embodiment of the present disclosure;
FIG. 3 is a schematic view of a wearable medical sensor system in accordance with a second preferred embodiment of the present disclosure;
FIG. 4 is a schematic view of a wearable medical sensor system in accordance with a third preferred embodiment of the present disclosure; and
FIG. 5 is a schematic view of a wearable medical sensor system in accordance with a fourth preferred embodiment of the present disclosure.

### DESCRIPTION OF THE PREFERRED EMBODIMENTS

The technical contents of the present disclosure will become apparent with the detailed description of preferred embodiments accompanied with the illustration of related drawings as follows. It is noteworthy that same numerals are used for representing same respective elements in the drawings.

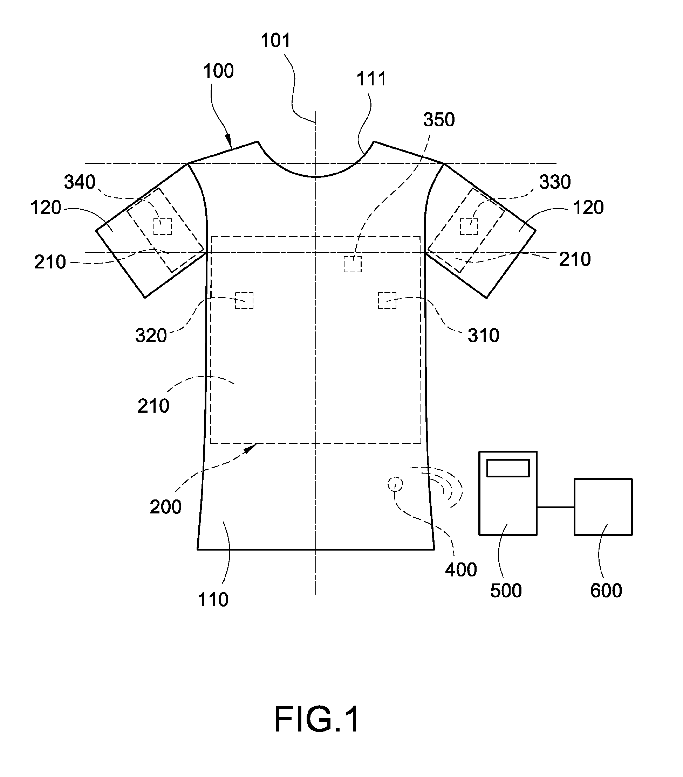

With reference to FIG. 1 for a wearable medical sensor system in accordance with the first preferred embodiment of the present disclosure, the wearable medical sensor system comprises a piece of clothes 100 and an elastic bounding layer 200 attached on an inner side of the piece of clothes 100.

The piece of clothes 100 includes a main body 110 and a pair of sleeves 120 connected to both sides of the main body 110 respectively. Preferably, the main body 110 is formed by connecting two pieces of fabrics and substantially in a rectangular shape, and the main body 110 is in a shape symmetric on both sides with respect to an axis of symmetry 101. One end of the main body 110 is an open end, and the other end has a collar 111. Each sleeve 120 is in a tubular shape, and the two sleeves 120 are disposed respectively and symmetrically on both sides of the main body 110 with respect to the axis of symmetry 101 and interconnected to the main body 110.

Preferably, the elastic bounding layer 200 is made of an elastic fabric with a contractility greater than the contractility of the clothes 100, and the elastic bounding layer 200 has a stretchability greater than the stretchability of the clothes 100. In addition, at least a part of the main body 110 is surrounded by the elastic bounding layer 200. In this preferred embodiment, the elastic bounding layer 200 includes three belt 210 made of elastic fabric, and these belts 210 are separated from each other and surrounded around the main body 110 and each sleeve 120.

A first electrode 310, a second electrode 320, a third electrode 330, a fourth electrode 340 and a fifth electrode 350 are attached on the elastic bounding layer 200 and disposed on the same surface of the clothes 100. In this preferred embodiment, the first electrode 310, the second electrode 320, the third electrode 330, the fourth electrode 340 and the fifth electrode 350 preferably are conductive fabrics, but the present disclosure is not limited to such arrangement only.

The first electrode 310 and the second electrode 320 are disposed symmetrically on opposite sides of the axis of symmetry 101 and arranged under a lower-edge connecting line of the junction of the main body 110 and each sleeve 120. The third electrode 330 and the fourth electrode 340 are arranged between an upper-edge connecting line and the lower-edge connecting line of the junction of the main body 110 and each sleeve 120. In this preferred embodiment, the third electrode 330 and the fourth electrode 340 are preferably and respectively disposed at the sleeves 120. The fifth electrode 350 is disposed at the lower-edge connecting line of the junction of the main body 110 and each sleeve 120 and configured to be biased towards the axis of symmetry 101.

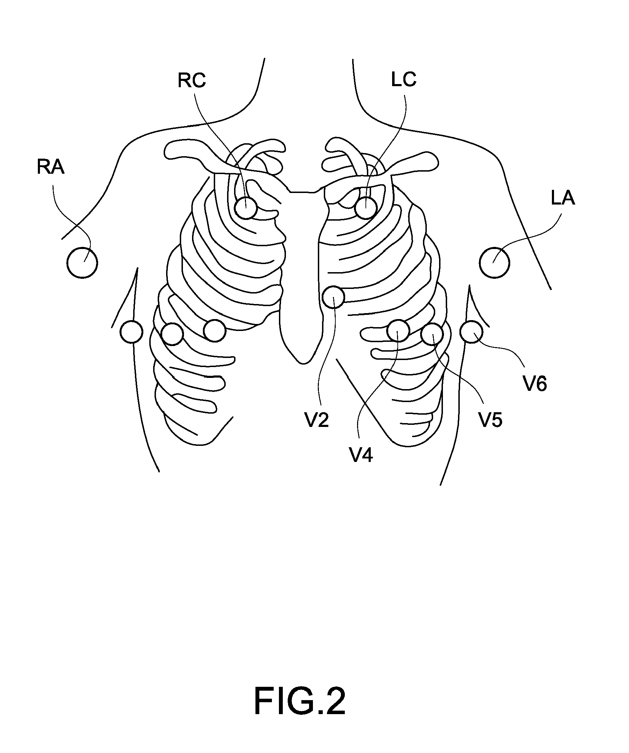

With reference to FIG. 2, when a user wears the wearable medical sensor system of the present disclosure, the elastic bounding layer 200 is at least covered on the surface of the user's chest. In this preferred embodiment, the belts 210 of the elastic bounding layer 200 are tied to the user's chest and arms, such that the first electrode 310, the second electrode 320, the third electrode 330, the fourth electrode 340 and the fifth electrode 350 corresponsive to the specific measuring positions of a human electrocardiographic system are fixed on the user's body. The first conductive fabric is disposed at any position of the connecting line of the fifth left intercostal space at the midclavcular line V4, the anterior axillary line V5 and the midaxillary line V6. The second conductive fabric is disposed at a position corresponsive to the right side of the first conductive fabric. The third conductive fabric is disposed at the horizontal upper left position of the heart, and preferably the left arm LA in this preferred embodiment. The fourth conductive fabric is disposed at the horizontal upper right position of the heart, and preferably the right arm RA in this preferred embodiment. The fifth conductive fabric is disposed at the fourth left intercostal space next to the sternum V2 of a human body. In this preferred embodiment, the belt 201 wrapped around the chest is separated from the belts 201 wrapped around the arms, so that when the user's shoulder or arm is moving, the belts 210 affect each other. In addition, chest is less easily affected than abdomen by movements in human structure, so that the belt 210 wrapped around the chest will not be dragged or pulled too much by human activities. In a movement of the human body, the shaking and pulling situations of the clothes 100 are occurred mostly in positions other than the belt 210, and thus the shaking and pulling situations of the belt 210 will be reduced. The area of the clothes other than the circular areas can maintain the characteristic of easy movement of a general piece of clothes. When the human body is moving, the first to fifth electrode 310/320/330/340/350 can be attached onto the skin, so as to provide a stable and tight attachment to the chest and arms and measure biological signals stably.

In FIG. 1, the piece of clothes 100 includes an ECG signal processing device 400 electrically coupled to the first electrode 310, the second electrode 320, the third electrode 330, the fourth electrode 340 and the fifth electrode 350. The ECG signal processing device 400 is capable of capturing an analog reference signal by any one of the first electrode 310, the second electrode 320, the third electrode 330, the fourth electrode 340 and the fifth electrode 350. The ECG signal processing device 400 is capable of capturing a plurality of analog differential signals by any two of the first electrode 310, the second electrode 320, the third electrode 330, the fourth electrode 340 and the fifth electrode 350. The ECG signal processing device 400 amplifies and filters the analog differential signals to generate a plurality of analog differential ECG signals, and converts each analog differential ECG signal into a digital differential ECG signal.

The ECG signal processing device 400 can transmit the differential ECG signals to a smart device 500 via wireless transmission. The smart device 500 receives the digital differential ECG signals and obtains the 1^{st} to 12^{th} lead digital ECG signals through a vector addition or subtraction operation. The smart device 500 further displays a portion or all of the digital ECG signals and records the digital ECG signals. The smart device 500 may be connected to another digital device 600 to transmit the recorded digital ECG signals.

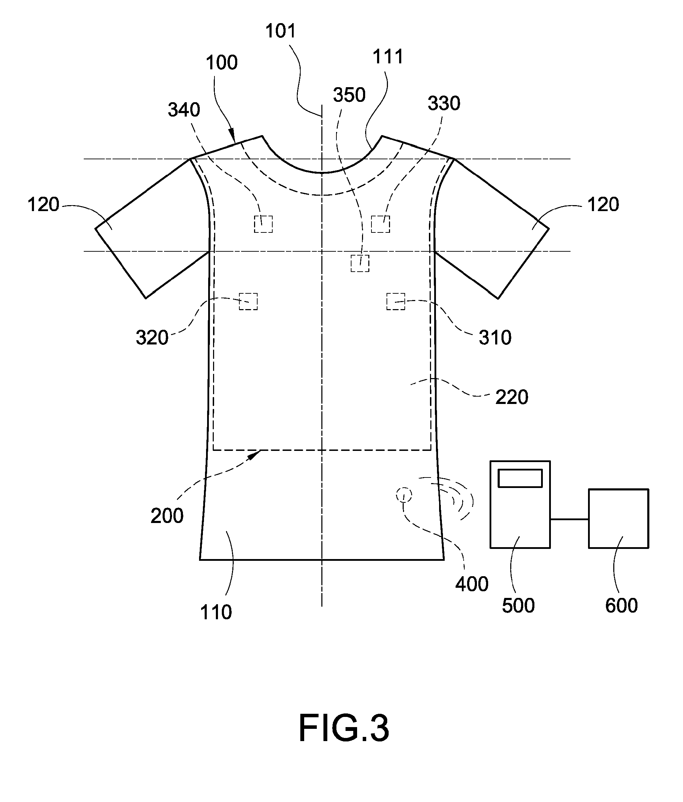

With reference to FIG. 3 for a wearable medical sensor system in accordance with the second preferred embodiment of the present disclosure, the wearable medical sensor system comprises a piece of clothes 100 and an elastic bounding layer 200 attached on an inner side of the clothes 100, and its structure is substantially the same as the first preferred embodiment, except that the elastic bounding layer 200 is attached on the vest 220 at the inner side of the main body 110, and the third electrode 330 and the fourth electrode 340 are preferably disposed at the main body 110. The vest 220 and the clothes 100 are just connected to both sides of the collar 111, so that when the user is moving, the shaking of the loose clothes 100 hardly affects or pulls the vest 220. Particularly, when the user's shoulder or arms are moving, the vest 220 is not affected by the sleeves 120 of the clothes 100 at all. The lower edge of the vest 220 may be wrapped to the human abdomen. Even if the junction of the vest 220 and the clothes 100 is pulled by the movement of the human body, the lower edge of the vest 220 still binds the human body, so as to reduce the impact of pulling. The elastic bounding layer 200 provides the effect of a stable and tight attachment to the chest, so that the first to fifth electrodes 310/320/330/340/350 can be attached onto skin stably to ensure a stable measurement of biological signals.

When a user wears the wearable medical sensor system of the present disclosure, the third conductive fabric is disposed at the horizontal upper left position of the user's heart, and preferably the user's left chest LC and on the left midclavcular line, and the fourth conductive fabric is disposed at the horizontal upper right position of the user's heart and preferably the user's right chest RC and on the right midclavcular line.

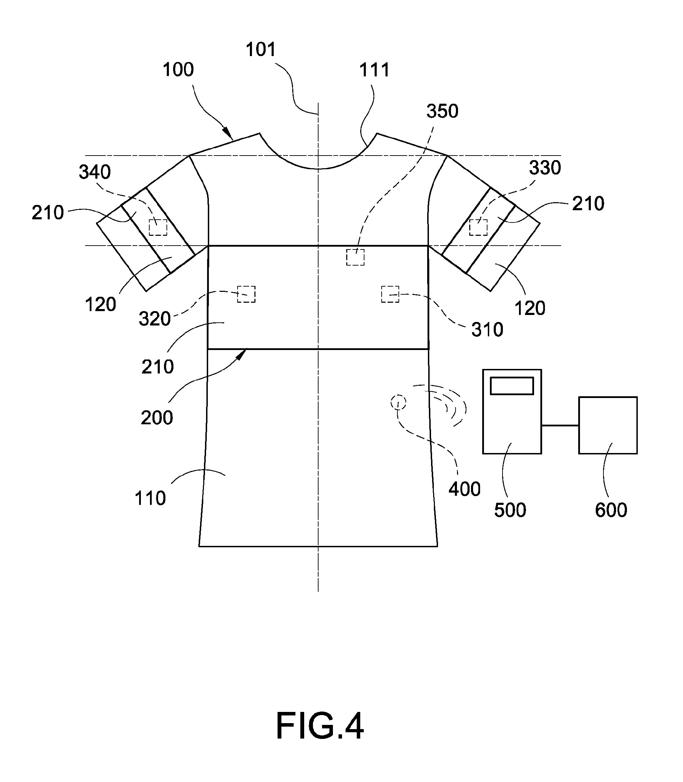

With reference to FIG. 4 for a wearable medical sensor system in accordance with the third preferred embodiment of the present disclosure, the wearable medical sensor system comprises a piece of clothes 100 and an elastic bounding layer 200, and its structure is substantially the same as the first preferred embodiment, except that the main body 110 is surrounded by at least a part of the elastic bounding layer 200. In this preferred embodiment, the elastic bounding layer 200 includes three belts spliced with the main body 110 and each sleeve 120. The elastic bounding layer 200 provides an effect of stably and tightly attaching a human body, so that the first to fifth electrode 310/320/330/340/350 can be attached onto a user's skin stably while the user is moving, so as to ensure the stable measurement of biological signals.

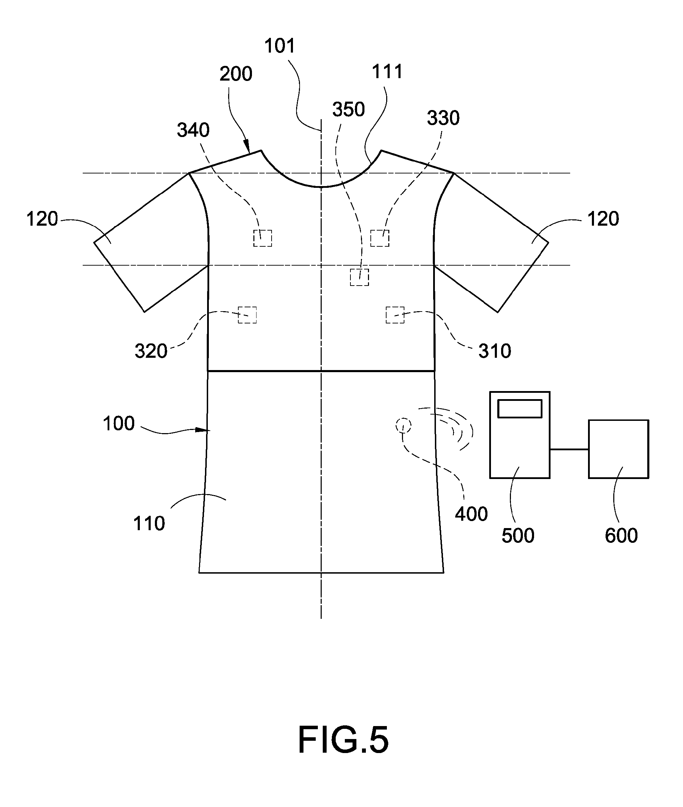

With reference to FIG. 5 for a wearable medical sensor system in accordance with the fourth preferred embodiment of the present disclosure, the wearable medical sensor system comprises a piece of clothes 100 and an elastic bounding layer 200 and its structure is substantially the same as the first preferred embodiment, except that the main body is surrounded by at least a part of the elastic bounding layer 200. In this preferred embodiment, the elastic bounding layer 200 is in the shape of a vest, and the elastic bounding layer 200 is spliced with the main body 110, and the third electrode 330 and the fourth electrode 340 are preferably disposed at the main body 110. The elastic bounding layer 200 provides an effect of a stable and tight attachment to human bodies, so that the first to fifth electrodes 310/320/330/340/350 can be attached onto a user's skin to ensure the stable measurement of biological signals while the user's body is moving.

In the wearable medical sensor system of the present disclosure, the elastic bounding layer 200 is connected to the electrodes and provided for fixing the electrodes at their relative positions. Therefore, when a user wears the clothes 100, the elastic bounding layer 200 fixes each electrode onto a predetermined measuring position of the user's body, so that the user can install the electrodes alone and the wearable medical sensor system of the present disclosure is suitable for long-term monitoring.

## Claims

1. A wearable medical sensor system, comprising a piece of clothes (100) and an elastic bounding layer (200) attached on an inner side of the piece of clothes (100), and the clothes (100) including a main body (110) with a shape symmetric on both sides with respect to an axis of symmetry (101) and a pair of sleeves (120) symmetrically connected to both sides of the main body (110), and at least a part of the main body (110) being surrounded by the elastic bounding layer (200), and a first electrode (310), a second electrode (320), a third electrode (330), a fourth electrode (340) and a fifth electrode (350) being attached on the elastic bounding layer (200) and disposed on the same surface corresponsive to the clothes (100), and the elastic bounding layer (200) having a contractility greater than the contractility of the clothes (100), and the elastic bounding layer (200) having a stretchability greater than the stretchability of the clothes (100).

2. The wearable medical sensor system of claim 1, wherein the first electrode (310), the second electrode (320), the third electrode (330), the fourth electrode (340) and the fifth electrode (350) are conductive fabrics.

3. The wearable medical sensor system of claim 1 or 2, wherein the first electrode (310) and the second electrode (320) are disposed symmetrically on both sides of the axis of symmetry (101) and arranged under a lower-edge connecting line of the junction of the main body (110) and each sleeve (120), and the third electrode (330) and the fourth electrode (340) are arranged between an upper-edge connecting line of the junction of the main body (110) with each sleeve (120) and the lower-edge connecting line and disposed on both sides of the axis of symmetry (101) respectively, and the fifth electrode (350) is disposed at the lower-edge connecting line of the junction of the main body (110) and each sleeve (120) and configured to be biased towards a side of the axis of symmetry (101).

4. The wearable medical sensor system of any of the claims 1 to 3, wherein the third electrode (330) and the fourth electrode (340) are configured to be corresponsive to the sleeves (120) respectively.

5. The wearable medical sensor system of any of the claims 1 to 4, wherein the elastic bounding layer (200) includes a belt (210) attached to the clothes (100).

6. The wearable medical sensor system of any of the claims 1 to 5, wherein the elastic bounding layer (200) includes a vest (220) attached on an inner side of the main body (110), and only a portion of the elastic bounding layer (200) is coupled to a portion of the clothes (100).

7. The wearable medical sensor system of any of the claims 1 to 6, wherein the elastic bounding layer (200) is attached on an inner side of the main body (110) and extended to an inner side of at least one of the sleeves (120).

8. The wearable medical sensor system of any of the claims 1 to 7, further comprising an ECG signal processing device (400) attached on the clothes (100) and electrically coupled to the first electrode (310), the second electrode (320), the third electrode (330), the fourth electrode (340) and the fifth electrode (350).

9. A wearable medical sensor system, comprising a piece of clothes (100), an elastic bounding layer (200) spliced with the clothes (100), a main body (110) with a shape symmetric on both sides with respect to an axis of symmetry (101), and a pair of sleeves (120) respectively and symmetrically coupled to both sides of the main body (110), and surrounded by at least a part of the elastic bounding layer (200), and a first electrode (310), a second electrode (320), a third electrode (330), a fourth electrode (340) and a fifth electrode (350) being attached on the elastic bounding layer (200) and installed on a same surface corresponsive to the clothes (100), and the elastic bounding layer (200) having a contractility greater than the contractility of the clothes (100), and the elastic bounding layer (200) having a stretchability greater than the stretchability of the clothes (100).

10. The wearable medical sensor system of claim 9, wherein the first electrode (310), the second electrode (320), the third electrode (330), the fourth electrode (340) and the fifth electrode (350) are conductive fabrics.

11. The wearable medical sensor system of claim 9 or 10, wherein the first electrode (310) and the second electrode (320) are disposed on both sides of the axis of symmetry (101) respectively and arranged under a lower-edge connecting line of the junction of the main body (110) and each sleeve (120), and the third electrode (330) and the fourth electrode (340) are arranged between the upper-edge connecting line and the lower-edge connecting line of the junction of the main body (110) and each sleeve (120) and disposed on both sides of the axis of symmetry (101) respectively, and the fifth electrode (350) is disposed on the lower-edge connecting line of the junction of the main body (110) and each sleeve (120) and configured to be biased towards a side of the axis of symmetry (101).

12. The wearable medical sensor system of any of the claims 9 to 11, wherein the third electrode (330) and the fourth electrode (340) are configured to be corresponsive to the sleeves (120) respectively.

13. The wearable medical sensor system of any of the claims 9 to 12, wherein the elastic bounding layer (200) includes a belt (210) spliced with the clothes (100).

14. The wearable medical sensor system of claim any of the claims 9 to 13, further comprising an ECG signal processing device (400) attached on the clothes (100) and electrically coupled to the first electrode (310), the second electrode (320), the third electrode (330), the fourth electrode (340) and the fifth electrode (350).
